# EUROPÄISCHE PATENTSCHRIFT

(11) **EP 2 757 088 B1**
(45) Veröffentlichungstag und Bekanntmachung des Hinweises auf die Patenterteilung: **03.08.2016**
(21) Anmeldenummer: 14151583.3
(22) Anmeldetag: 17.01.2014
(51) Int. Cl.: C07C 209/36, C07C 211/50

(54) **Verfahren zur Herstellung von aromatischen Aminen**
Method for manufacturing aromatic amines
Procédé destiné à la fabrication d'amines aromatiques

(30) Priorität: 22.01.2013 EP 13152196
(43) Veröffentlichungstag der Anmeldung: 23.07.2014
(73) Patentinhaber: Covestro Deutschland AG, 51373 Leverkusen (DE)
(72) Erfinder: Pennemann, Bernd, 51467 Bergisch Gladbach (DE); Temme, Bodo, 41542 Dormagen (DE)
(74) Vertreter: Levpat

(56) Entgegenhaltungen:
- WO-A1-03/066571
- WO-A1-2012/076449
- WO-A2-02/062729
- WO-A2-2011/086050

## Beschreibung

Die Erfindung betrifft ein Verfahren zur Herstellung von organischen Aminoverbindungen aus organischen Nitroverbindungen, wobei die organische Nitroverbindung zu der organischen Aminoverbindung mit einem Wasserstoff-haltigen Gasstrom mittels eines Katalysators hydriert wird, wobei der Reaktionsverlauf der Hydrierung durch die Analyse von bei der Hydrierung entstehenden Nebenprodukten überwacht wird.

Die Herstellung organischer Aminoverbindungen aus entsprechenden Nitroverbindungen ist aus dem Stand der Technik hinlänglich bekannt. Bei dem in EP 0 223 035 A1 beschriebenen Verfahren wird die Hydrierung mit Hilfe von im flüssigen Reaktionsgemisch dispergierten modifizierten Raney-Nickel-Katalysatoren durchgeführt. Der Katalysator kann aus dem flüssigen Reaktionsgemisch durch Filtration oder Sedimentation abgetrennt und gegebenenfalls dem Prozess wieder zugeführt werden.

Weiterhin ist aus DE 2044657 ein Verfahren zur Herstellung von Toluylendiamin durch Hydrierung von Dinitrotoluol bei erhöhtem Druck und erhöhter Temperatur an einem oder zwei hintereinandergeschalteten Reaktoren bekannt. Als Reaktoren werden hier Hochdruckrohre mit darin fest angeordneten Nickel- oder Ruthenium-Hydrierkatalysatoren vorgeschlagen.

Organische Aminoverbindungen, insbesondere Di- oder Triaminoverbindungen, stellen einen wichtigen Ausgangsstoff für die Herstellung von organischen Polyisocyanaten dar, die wiederum für die Herstellung von Polyurethanen benötigt werden. Hierzu wird beispielsweise ausgehend von Dinitrotoluol über eine Hydrierung Toluylendiamin (TDA) erzeugt, welches ein häufig verwendetes Monomer zur Erzeugung von Polyisocyanaten darstellt. Für die Hydrierung werden typischerweise Katalysatoren eingesetzt, wobei die Hydrierung in flüssiger Phase durchgeführt wird. Dabei können im laufenden Produktionsprozess beispielsweise durch unzureichende Katalysatoraktivität Probleme derart auftauchen, dass im Reaktor eine Akkumulation von Dinitrotoluol stattfindet. Diese Akkumulation stellt ein erhebliches Sicherheitsrisiko dar, da sich Dinitrotoluol bei erhöhter Temperatur und insbesondere in Gegenwart von starken Basen explosionsartig zersetzen kann. Um dieses Sicherheitsrisiko einzudämmen ist es ein kontinuierliches Ziel, einen vollständigen Umsatz des dem Hydrierreaktor zugegebenen Dinitrotoluol sicherzustellen. Hierzu sind aus dem Stand der Technik mehrere Ansätze bekannt.

Aus WO 03/066571 A1 ist es bekannt, die Vollständigkeit der Hydrierungsreaktion durch gaschromatographische Analyse des flüssigen Hydrierungsproduktes zu vollziehen.

In DE 10 2005 008 613 A1 ist vorgeschlagen, eine Bestimmung der Konzentration an Nitro-und Nitrosoverbindungen über UV / VIS absorptionsspektroskopische Methoden vorzunehmen.

WO 2012/076449A1 beschreibt ein Verfahren zur Herstellung von aromatischen Aminen durch Hydrierung von Nitroaromaten, bei dem eine chromatographische Analyse des Reaktionsgemisches zur Konzentrationsbestimmung von Nitro- und Nitrosoverbindungen in dem Reaktionsgemisch durchgeführt wird.

Darüber hinaus können erfahrene Anlagenbetreiber auch aus der Farbe des Hydrierproduktes Rückschlüsse auf den Reaktionsverlauf ziehen. Daneben ist es bekannt, dass die Vollständigkeit der Reaktion auch durch eine Überwachung des Temperaturanstiegs in einem dem eigentlichen Reaktor nachgeschalteten Reaktionsraum erfolgen kann. Zudem kann eine Unvollständigkeit der Reaktion auch an einer Abweichung der Reaktionsstöchiometrie, konkret durch Untersuchung des Verhältnisses zwischen Wasserstoff und Dinitrotoluol erkannt werden. Diese Methoden sind beispielsweise in WO 2011/086050A2 beschrieben.

Die bislang aus dem Stand der Technik bekannten Maßnahmen zur Überprüfung der Vollständigkeit der Hydrierreaktionen sind nicht in jeder Hinsicht zufriedenstellend. So sind diese Methoden zum Teil mit erhöhtem apparativem Aufwand verbunden oder teilweise der Erfahrung des Bedienungspersonals überlassen, was naturgemäß zu Fehlern führen kann. Andere Methoden, wie beispielsweise die Bestimmung des Temperaturanstiegs liefern die erforderlichen Daten zeitlich verzögert, was ebenfalls nachteilig für eine optimale Steuerung des Hydrierreaktors ist.

Die Aufgabe der vorliegenden Erfindung bestand somit darin, ein Verfahren zur Herstellung organischer Amine durch Hydrierung entsprechender Nitroverbindungen zur Verfügung zu stellen, welches sich mit vergleichsweise geringem apparativem Aufwand verwirklichen lässt und dabei zeitnah Informationen über die Vollständigkeit der Hydrierungsreaktion liefert. Dabei soll das Verfahren sicher in der Bedienung sein und vor allem eine quantitative Umsetzung der organischen Nitroverbindung bei gleichzeitiger Optimierung der Ausbeute an organischer Aminoverbindung ermöglichen.

Die Aufgabe wird gelöst durch ein Verfahren zur Herstellung von organischen Aminoverbindungen aus organischen Nitroverbindungen, wobei die organische Nitroverbindung zu der organischen Aminoverbindung mit einem Wasserstoff-haltigen Gasstrom mittels eines Katalysators hydriert wird, wobei der Reaktionsverlauf der Hydrierung durch die Analyse von bei der Hydrierung entstehenden Nebenprodukten überwacht wird, wobei das Verfahren dadurch gekennzeichnet ist, dass die Konzentration eines oder mehrerer gasfönniger Nebenprodukte in der Gasphase bestimmt und bei Unterschreiten einer vorgebbaren Mindestkonzentration des einen oder der mehreren gasförmigen Nebenprodukte die Hydrieraktivität des Katalysators erhöht wird. Unter *Hydrieraktivität des Katalysators* wird erfindungsgemäß die Fähigkeit des Katalysators verstanden, die Hydrierung organischer Nitroverbindungen zu organischen Aminoverbindungen zu katalysieren. Diese Hydrieraktivität kann gesteigert werden, indem ein oder mehrere Reaktionsparameter (Volumenstrom des Wasserstoff-haltigen Gasstroms, Menge der zugeführten organischen Nitroverbindung, Druck, Temperatur und/oder Verweilzeit) so geändert wird/werden, dass der in der Reaktionskammer vorhandene Katalysator die Hydrierung der organischen Nitroverbindung mit im Vergleich zum Zustand vor Änderung des/der Parameter(s) gesteigerter Aktivität katalysiert. Alternativ oder zusätzlich dazu kann auch weiterer Katalysator zugegeben werden, sodass der dann in der Reaktionskammer befindliche Katalysator, der ein Gemisch aus zuvor schon vorhandenem und frischem Katalysator ist, die Hydrierung der organischen Nitroverbindung mit im Vergleich zum Zustand vor der Zugabe weiteren Katalysators gesteigerter Aktivität katalysiert.

Bei der Hydrierung organischer Nitroverbindungen soll bei dem erfindungsgemäßen Verfahren zweckmäßigerweise immer eine solche Menge an Wasserstoff eingesetzt werden, dass die Hydrierung der Nitroverbindung vollständig abläuft, um eine aus Sicherheitsgründen problematische Akkumulation von organischer Nitroverbindung im Reaktor durch nicht abreagierte Nitroverbindung zu verhindern. Durch den zweckmäßigerweise überstöchiometrischen Einsatz von Wasserstoff entstehen neben der gewünschten organischen Amninoverbindung Nebenprodukte. So können beispielsweise beim Einsatz aromatischer Nitroverbindungen auch kernhydrierte Aminoverbindungen als Nebenprodukte entstehen.

Der Erfindung liegt nun die Erkenntnis zugrunde, dass anhand einer Analyse von Nebenprodukten in dem aus dem Hydrierreaktor ausgeschleustem Gasstrom eine Aussage über die Vollständigkeit der Hydrierreaktion getätigt werden kann. Bei der vorliegenden Erfindung wird mit anderen Worten die an sich unerwünschte Unselektivität der Hydrierungsreaktion ausgenutzt, um sicherzustellen, dass die gewünschte Hydrierreaktion stets möglichst vollständig abläuft, wobei vorteilhafterweise das untersuchte gasförmige Nebenprodukt Methan, Ammoniak und/ oder ein aliphatisches Amin ist, welches selbst nicht zu den Produkten zählt, wie beispielweise Methylamin oder Methylcyclohexylamin.

Die Hydrierung der organischen Nitroverbindung zum entsprechenden Amin erfolgt in der Regel mit Wasserstoff oder Gemischen aus Wasserstoff und inerten Gasen als Hydrierreagenz. Als Katalysatoren kommen dabei alle für katalytische Hydrierungen üblichen in Frage. Bevorzugt werden Katalysatoren, die Edelmetalle wie Pt, Pd, Rh, Ru oder Buntmetalle wie Ni, Co oder Cu oder deren Mischungen enthalten, eingesetzt. Besonders bevorzugt werden Katalysatoren, die Pt, Pd, Ni oder Cu enthalten, eingesetzt, und zwar in der Regel als Suspension in Wasser. Im Falle von Edelmetallkatalysatoren werden diese auf Träger wie bspw. Aktivkohle, SiO₂ oder Al₂O₃ aufgebracht, im Fall von Ni-Katalysatoren kann auch Raney-Ni eingesetzt werden. Die Konzentration von Katalysator im Reaktionsraum liegt bevorzugt bei 0,01 Gew.-% bis < 20 Ges.-%, bevorzugt bei 0,5 Gew.-% bis 10 Gew.-%, bezogen auf das Gesamtgewicht des Reaktionsgemisches im Reaktionsraum. Von den in Frage kommenden Katalysatoren wird man regelmäßig einen auswählen, der hinsichtlich des Zielproduktes besonders selektiv ist was auch bedingt, dass dieser möglichst geringe Mengen an Nebenprodukten erzeugt. Das schränkt die vorliegende Erfindung nicht ein, bedeutet jedoch, dass vorteilhafterweise die Nachweisgrenze für die Nebenprodukte entsprechend niedrig gewählt werden sollte.

Werden Gemische aus Wasserstoff und inerten Gasen eingesetzt, so sind bevorzugte Inertgase Ammoniak, Edelgase und/oder Stickstoff. Wasserstoff bzw. das Gemisch aus Wasserstoff und inerten Gasen wird bevorzugt so aufgegeben, dass sich im Reaktionsraum ein konstanter Druck einstellt, d. h. mit zunehmendem Reaktionsfortschritt (und damit Wasserstoffverbrauch) wird die Zufuhr von frischem Hydrierreagenz erhöht. Im Falle der Verwendung eines Gemisches aus Wasserstoff und inerten Gasen als Hydrierreagenz wird bevorzugt das Verhältnis aus Wasserstoff und Inertgas im zugeführten Hydrierreagenz sukzessive erhöht, um den Reaktorinhalt nicht an Wasserstoff verarmen zu lassen.

Bei dem erfindungsgemäßen Verfahren wird Wasserstoff vorzugsweise im Überschuss in Bezug auf die Hydrierung der Nitrogruppen zu Aminogruppen benötigte Menge eingesetzt. Insbesondere beträgt der Überschuss an Wasserstoff wenigstens 0,01 mol.-%, bevorzugt wenigstens 0,1 mol.-%, jeweils bezogen auf die für die Hydrierung der Nitrogruppen zu Aminogruppen benötigte Stoffmenge.

Optional können unter den Reaktionsbedingungen inerte Lösungsmittel eingesetzt werden, wie z. B. Alkohole wie Methanol, Propanol, Isopropanol oder Ether wie Dixoan, Tetrahydrofuran. Um die Wirtschaftlichkeit des Verfahrens zu erhöhen, ist in der Regel eine niedrige Lösungsmittelkonzentration vorteilhaft. Diese liegt üblicherweise bei 1 Gew.-% bis <50Gew.-%, bevorzugt bei 20 Gew.-% bis 35 Gew.-%, jeweils bezogen auf das Gesamtgewicht der Flüssigphase.

Weiterhin kann bei dem erfindungsgemäßen Verfahren 0,1 bis 10 Ges.- %, bevorzugt 0,2 bis 5 Gew.- % des eingesetzten Wasserstoffs ausgeschleust werden und darin die Konzentration des einen oder der mehreren gasförmigen Nebenprodukte bestimmt werden. Das ist insbesondere von Vorteil, da es apparativ unkomplizierter ist, die Konzentrationsmessung in diesem Teilgasstrom vorzunehmen, als in der Produktleitung. Zudem kann durch diese Ausschleusung die Akkumulation von inerten Verbindungen, die beispielsweise als Spuren im zugeführten Wasserstoff enthalten sein können, verhindert werden.

Das erfindungsgemäße Verfahren wird bevorzugt kontinuierlich ausgeführt.

Erfindungsgemäß ist vorgesehen, dass die Konzentration des einen oder der mehreren gasförmigen Nebenprodukte in der Gasphase bestimmt wird. Vorzugsweise besitzen die untersuchten gasförmigen Nebenprodukte einen Siedepunkt bei Normaldruck von 200 °C oder weniger, insbesondere von 180 °C oder weniger, weiter bevorzugt von 80 °C oder weniger, besonders bevorzugt von 50 °C oder weniger, ganz besonders bevorzugt von 20 °C oder weniger.

Die Mindestkonzentration des einen oder der mehreren gasförmigen Nebenprodukte in der Gasphase kann je nach untersuchtem Nebenprodukt variieren. Die im Folgenden angegebenen Mindestkonzentrationen beziehen sich aufgrund der Variabilität des ausgeschleusten Volumenstroms, insbesondere des ausgeschleusten gasförmigen Wasserstoffs, auf die Menge an eingesetzter organischer Nitroverbindung. Diese kann beispielsweise aus der Volumenkonzentration an Nebenprodukt im ausgeschleusten Wasserstoff, dem Volumenstrom des ausgeschleusten Wasserstoffs, dem Druck, dem Molgewicht des Nebenprodukts sowie an der Menge an zugesetzter organischer Nitroverbindung, bestimmt werden.

Im Fall von Methan und Ammoniak beträgt die Mindestkonzentration beispielsweise wenigstens 2 mg / kg organischer Nitroverbindung, bevorzugt 2 bis 20 mg / kg, insbesondere 2 mg / kg, vorzugsweise 4 mg / kg, weiter bevorzugt 10 mg / kg, oder gar 15 mg / kg, im Falle des aliphatischen Amins beispielsweise wenigstens 2 mg / kg organischer Nitroverbindung, bevorzugt 1 bis 10 mg / kg, insbesondere 1 mg / kg, vorzugsweise 2 mg / kg, weiter bevorzugt 5 mg / kg, oder gar 7,5 mg / kg. Die exakten Werte können von Katalysatortyp und Reaktionsbedingungen abhängen und falls gewünscht noch angepasst werden. Dies kann beispielsweise durch gezielte Korrelation mit einem oder mehreren der Verfahren zur Überprüfung der Vollständigkeit der Reaktion nach dem Stande der Technik erfolgen. Derartige Verfahren sind dem Fachmann an sich bekannt. Von den vorgenannten Nebenprodukten ist die Bestimmung von Methan besonders vorteilhaft, da dieses beispielsweise über eine IR-spektroskopische Untersuchung leicht nachweisbar ist. Das Methan entsteht beispielsweise bei der Hydrierungsreaktion von Dinitrotoluol durch Abspaltung der Methylgruppe vom aromatischen Ring.

Bei dem erfindungsgemäßen Verfahren können zur Bestimmung der Konzentration des gasförmigen Nebenprodukts im Prinzip sämtliche dem Fachmann hierfür bekannte Methoden zum Einsatz kommen. Besonders geeignet ist hierfür ist ein Gaschromatograph oder eine Gas-Sonde, wie beispielsweise eine geeignete Gas-Elektrode. Ebenso können auch alternativ oder zusätzlich spektroskopische Methoden zum Einsatz kommen, insbesondere ein UV / VIS- und / oder IR-Spektrometer.

Die Vorrichtung zur Bestimmung der Konzentration des gasförmigen Nebenprodukts kann im Prinzip an jeder geeigneten Stelle angebracht sein. Beispielsweise kann die Vorrichtung an eine Leitung angeschlossen oder aber auch im Reaktor angeordnet sein.

Nach einer bevorzugten Ausführungsform des erfindungsgemäßen Verfahrens wird vor der Bestimmung der Konzentration des gasförmigen Nebenprodukts in der Gasphase enthaltener Wasserdampf durch Kondensation entfernt. Dies ist besonders vorteilhaft, da Wasserdampf möglicherweise die Bestimmung des Nebenproduktes erschwert, insbesondere bei einer IRspektroskopischen Untersuchung.

Weiterhin kann erfindungsgemäß vorgesehen sein, dass bei dem Verfahren das gasförmige Nebenprodukt vor der Konzentrationsbestimmung durch eine Trenneinrichtung geführt wird, insbesondere durch eine chromatographische Säule, vorzugsweise eine GC-Säule. Auf diese Weise können möglicherweise störende Produkte abgetrennt werden, wodurch sich die Bestimmungsgenauigkeit desjenigen Nebenproduktes verbessert, welches zur Überwachung des Reaktionsverlaufs der Hydrierung herangezogen wird.

Die erfindungsgemäß vorgesehene Erhöhung der Hydrieraktivität des Katalysators erfolgt durch eine Erhöhung des Volumenstroms des Wasserstoff-haltigen Gasstroms, durch Drosselung der Zuführung der organischen Nitroverbindung, durch eine Erhöhung des Drucks, durch eine Erhöhung der Temperatur, durch eine Erhöhung der Verweilzeit und / oder durch Zugabe weiteren Katalysators.

Nach einer weiteren bevorzugten Ausgestaltung des erfindungsgemäßen Verfahrens wird verbrauchter Katalysator kontinuierlich oder schrittweise aus dem Reaktionsraum ausgeschleust und ggf. durch frischen Katalysator ersetzt.

Die bei dem erfindungsgemäßen Verfahren eingesetzte organische Nitroverbindung kann auf jede dem Fachmann bekannte Weise hergestellt sein. Typischerweise wird die organische Nitroverbindung durch Nitrierung einer organischen Verbindung insbesondere mit NOₓ und/ oder Salpetersäure erzeugt. Die organische Verbindung ist dabei ausgewählt aus den aromatischen Kohlenwasserstoffen Benzol und mit Methyl und/oder Ethyl substituierten Benzolen, insbesondere Toluol. Das erfindungsgemäße Verfahren betrifft demnach die Herstellung von aromatischen Aminen aus aromatischen Nitroverbindungen, insbesondere von aromatischen Poly-Aminen aus aromatischen Poly-Nitroverbindungen.

Erfindungsgemäß handelt es sich bei der verfahrensgemäß eingesetzten organischen Nitroverbindung um einen Nitroaromaten der Formel (I) 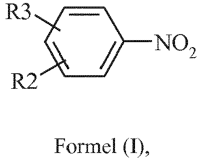 in der R2 und R3 unabhängig voneinander Wasserstoff, Methyl oder Ethyl bedeuten, wobei R3 zusätzlich NO₂ bedeuten kann.

Bei der organischen Aminoverbindung handelt es sich im Rahmen des erfindungsgemäßen Verfahrens um ein aromatisches Amin der Formel (II) 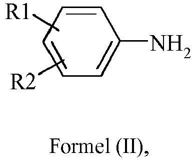 in der R1 und R2 unabhängig voneinander Wasserstoff, Methyl oder Ethyl bedeuten, wobei R1 zusätzlich NH₂ bedeuten kann.

Bei dem erfindungsgemäßen Verfahren kann die Hydrierung bei dem hierfür dem Fachmann geläufigen Temperaturen und Drücken erfolgen, wobei bevorzugte Temperaturen im Bereich von 80 bis 200 °C liegen, insbesondere bei 110 bis 180 °C sowie bevorzugte Drücke bei 5 bis 120 bar, insbesondere bei 10 bis 100 bar. Die Temperaturmessung erfolgt mittels dem Fachmann bekannter Vorrichtungen, wie Thermoelemente oder Widerstands-, Halbleiter- oder Infrarotthermometer. Die Druckmessung erfolgt vorzugsweise durch mechanische Manometer oder elektronische Drucksensoren.

Eine für die Durchführung des erfindungsgemäßen Verfahrens geeignete Vorrichtung ist eine Vorrichtung zur Herstellung von organischen Aminoverbindungen, umfassend eine Reaktionskammer zur Hydrierung einer organischen Nitroverbindung mittels eines Katalysators und eine Einrichtung zur Überwachung des Reaktionsverlaufs der Hydrierung durch Analyse von bei der Hydrierung entstehenden Nebenprodukten, wobei die Vorrichtung dadurch gekennzeichnet ist, dass die Einrichtung zur Überwachung geeignet ist, die Konzentration gasförmiger Nebenprodukte in der Gasphase zu bestimmen und ferner mit einer Steuerungseinrichtung gekoppelt ist, die bei Unterschreiten einer vorgebbaren Mindestkonzentration des oder der Nebenprodukte die Hydrieraktivität des Katalysators erhöht.

Unter einer Reaktionskammer wird ein Raum verstanden, in welchem die Voraussetzungen für eine Reaktion in der Flüssigphase von Nitroaromat (bzw. Zwischenprodukten) mit Wasserstoff zum gewünschten aromatischen Amin gegeben sind. Die Reaktionskammer befindet sich in einer technischen Vorrichtung zur Durchführung von chemischen Reaktionen, dem Reaktor. Reaktionskammer und Reaktor können, je nach Konstruktion, auch identisch sein (z. B. bei Blasensäulenreaktoren). Die Reaktionskammer kann auch nur einen Teil des Reaktors umfassen. Wenn etwa nur im unteren Bereich eines Reaktors eine Flüssigphase vorliegt, so gehört die darüber liegende Gasphase nicht mehr zum Reaktionsraum, ungeachtet der Tatsache, dass infolge des Dampfdrucks des Nitroaromaten, möglicherweise ein - untergeordneter - Anteil des Nitroaromaten in die Gasphase eintritt und dort reagiert. Ein Reaktor kann auch mehrere Reaktionskammern enthalten. Die Reaktionskammern können sich in einem oder in verschiedenen Reaktoren befinden. Bevorzugte Reaktoren für das erfindungsgemäße Verfahren sind gerührte Kessel, Schlaufenreaktoren, Strömungsrohre, Blasensäulenreaktoren oder Jet-Reaktoren.

Bei Verwendung mehrerer Reaktionskammern sind diese im erfindungsgemäßen Verfahren bevorzugt hintereinandergeschaltet, d. h. die Produktmischung aus einer Reaktionskammer wird in die folgende Reaktionskammer als Eduktmischung eingeleitet. Es ist möglich, jedoch nicht unbedingt zwingend erforderlich, in die nachgeschalteten Reaktionskammern zusätzlich frischen Wasserstoff oder ein Gemisch aus Wasserstoff und Inertgasen und ggf. frischen Katalysator einzuleiten. Im erfindungsgemäßen Verfahren wird der frische Nitroaromat typischerweise nur in eine Reaktionskammer eingeleitet; dieser wird als der in Anströmrichtung des Nitroaromaten erste bezeichnet und bevorzugt isotherm betrieben. Alle nachfolgenden Reaktionskammern werden nur mit Nitroaromaten, welcher in der jeweils vorigen Reaktionskammer nicht umgesetzt wurde, belastet. Demnach werden bei Vollumsatz in der in Anströmrichtung des Nitroaromaten ersten Reaktionskammer die nachfolgenden überhaupt nicht mit Nitroaromaten belastet.

In vorteilhafter Ausgestaltung der erfindungsgemäßen Vorrichtung ist die Einrichtung zur Überwachung zur Bestimmung der Konzentration gasförmiger Nebenprodukte über eine Analysegasleitung mit der Reaktionskammer verbunden.

Weiterhin kann bei der Vorrichtung vorgesehen sein, dass der Einrichtung zur Überwachung eine Kondensationseinrichtung zur Kondensation von Wasserdampf vorgeschaltet ist. Durch diese apparative Ausgestaltung kann Wasserdampf vor der quantitativen Bestimmung des Nebenproduktes aus dem Gasstrom entfernt werden, welcher möglicherweise eine exakte mengenmäßige Bestimmung des Nebenproduktes erschweren könnte. Dies ist insbesondere vorteilhaft, wenn die Bestimmungen der Konzentration der gasförmigen Nebenprodukte mittels eines IR-Spektrometers erfolgt.

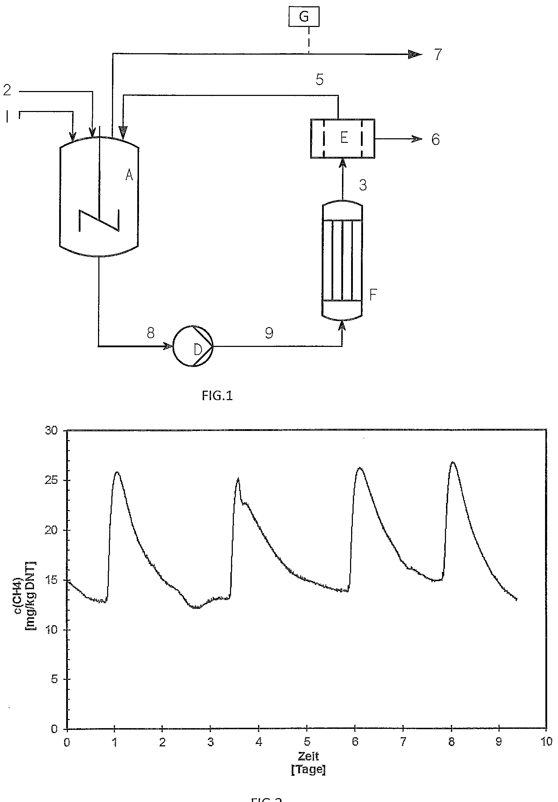

Die Erfindung wird im Folgenden anhand eines Ausführungsbeispiels und der Fign. 1 und 2 näher erörtert. Darin zeigt
- Fig. 1: den schematischen Aufbau einer Vorrichtung zur Durchführung des erfindungsgemäßen Verfahrens, sowie
- Fig.2: den zeitlichen Verlauf der gemessenen Methankonzentration im ausgeschleusten Gasstrom.

### Beispiel:

In Fig. 1 ist ein Reaktor A dargestellt. Dem Reaktor A wird geschmolzenes Dinitroluol über eine Eduktleitung 1 sowie Wasserstoff über eine Wasserstoffleitung 2 zugeführt. In dem Reaktor A befindet sich ein Gemisch aus Toluylendiamin und Wasser sowie einem NickelKatalysator, wobei das Dinitrotoluol bei einer Temperatur von 130°C und einem Druck von 20 bar mittels Wasserstoff hydriert wird.

Aus dem Reaktor A wird mittels einer Pumpe D über Teilleitungen 8, 9, 3, 5 ein Teil des Reaktionsgemischs umfassend unter anderem das Edukt, das Produkt und den Katalysator im Kreislauf befördert. Die Teilleitung 9 mündet in einen Kühler F, in welchem das Reaktionsgemisch abgekühlt wird. Von dort wird das abgekühlte Reaktionsgemisch über die Teilleitung 3 einem Querstromfilter E zugeführt, in dem das Hydrierungsprodukt, also Diaminotoluol, über eine Produktleitung 6 abgeführt wird. Der Rest des Reaktionsgemischs wird über die Teilleitung 5 wieder dem Reaktor A zugeführt, womit der Kreislauf geschlossen ist.

Die Menge der Dinitrotoluolschmelze wird so eingestellt, dass sich eine mittlere Verweilzeit im Reaktor A von 2 h ergibt. Die dem Reaktor A über die Wasserstoffleitung 2 zugeführte Wasserstoffmenge wird so eingestellt, dass der Druck im Reaktor A konstant bleibt.

Der Reaktor A ist ferner über eine Leitung 7 mit einer photometrischen Messzelle G verbunden. Aus dem Reaktor A werden kontinuierlich 1% des zugegebenen Wasserstoffs zur Vermeidung der Akkumulation von Inerten über die Leitung 7 ausgeschleust. Zugleich wird mit Hilfe der photometrischen Messzelle G der Methangehalt im ausgeschleusten Wasserstoff quantitativ bestimmt. Es wird beispielsweise ein Methangehalt von etwa 12 mg/kg DNT festgestellt, was der vorgegebenen Mindestkonzentration an diesem Nebenprodukt entspricht.

Nach einem Tag wird eine Katalysatormenge entsprechend 20% der Ausgangsmenge dem Reaktor zugegeben, wobei der Methangehalt im ausgeschleusten Wasserstoff auf 25 mg/kg DNT ansteigt. In einem Zeitraum von ca. 2,5 weiteren Tagen sinkt der Methangehalt infolge Desaktivierung des Katalysators auf den Ausgangswert ab. Es wird erneut die gleiche Menge Katalysator zugegeben, entsprechend steigt der Methangehalt wieder auf 25 mg/kg DNT.-% an. Dieser zeitliche Verlauf ist in Fig. 2 dargestellt.

Mithilfe des erfindungsgemäßen Verfahrens kann also durch Bestimmung des Methangehaltes und Erhöhung der Hydrieraktivität des Katalysators die Ausbeute an organischer Aminoverbindung kontinuierlich auf einem hohen Niveau gehalten werden unter gleichzeitiger Einhaltung einer hohen Betriebssicherheit.

## Patentansprüche

1. Verfahren zur Herstellung von organischen Aminoverbindungen der Formel (II) 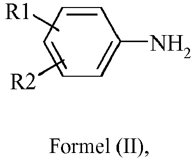 in der R1 und R2 unabhängig voneinander Wasserstoff, Methyl oder Ethyl bedeuten, wobei R1 zusätzlich NH₂ bedeuten kann, aus organischen Nitroverbindungen der Formel (I) 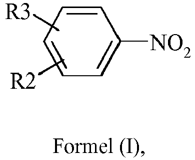 in der R2 und R3 unabhängig voneinander Wasserstoff, Methyl oder Ethyl bedeuten, wobei R3 zusätzlich NO₂ bedeuten kann, wobei die organische Nitroverbindung zu der organischen Aminoverbindung mit einem Wasserstoff-haltigen Gasstrom mittels eines Katalysators hydriert wird, wobei der Reaktionsverlauf der Hydrierung durch die Analyse von bei der Hydrierung entstehenden Nebenprodukten überwacht wird,
**dadurch gekennzeichnet, dass**
die Konzentration eines oder mehrerer gasförmige Nebenprodukte in der Gasphase bestimmt und bei Unterschreiten einer vorgebbaren Mindestkonzentration des einen oder der mehreren gasförmigen Nebenprodukte die Hydrieraktivität des Katalysators erhöht wird durch eine Erhöhung des Volumenstroms des Wasserstoff-haltigen Gasstroms, durch Drosselung der Zuführung der organischen Nitroverbindung, durch eine Erhöhung des Drucks, durch eine Erhöhung der Temperatur, durch eine Erhöhung der Verweilzeit und/ oder durch Zugabe weiteren Katalysators.

2. Verfahren nach Anspruch 1, **dadurch gekennzeichnet, dass** das gasförmigen Nebenprodukt einen Siedepunkt bei Normaldruck von 200 °C oder weniger aufweist, insbesondere von 180 °C oder weniger, weiter bevorzugt von 80 °C oder weniger, besonders bevorzugt von 50 °C oder weniger, ganz besonders bevorzugt von 20 °C oder weniger.

3. Verfahren nach Anspruch 1 oder 2, **dadurch gekennzeichnet, dass** das gasförmige Nebenprodukt Methan, Ammoniak und/ oder ein aliphatisches Amin ist, welches selbst nicht zu den Produkten zählt, wie Methylamin und/ oder Methylcyclohexylamin, wobei die bevorzugte Mindestkonzentration des gasförmigen Nebenprodukts
im Falle von Methan wenigstens 2 mg / kg organischer Nitroverbindung beträgt, bevorzugt 2 bis 20, insbesondere 2 mg / kg, vorzugsweise 4 mg / kg, weiter bevorzugt 10 mg / kg, oder gar 15 mg / kg,
im Falle von Ammoniak wenigstens 2 mg / kg organischer Nitroverbindung beträgt, bevorzugt 2 bis 20 mg / kg, insbesondere 2 mg / kg, vorzugsweise 4 mg / kg, weiter bevorzugt 10 mg / kg, oder gar 15 mg / kg, sowie
im Falle des aliphatischen Amins wenigstens 1 mg / kg organischer Nitroverbindung beträgt, bevorzugt 1 bis 10 mg / kg, insbesondere 1 mg / kg, vorzugsweise 2 mg / kg, weiter bevorzugt 5 mg / kg, oder gar 7,5 mg / kg.

4. Verfahren nach einem der vorstehenden Ansprüche, **dadurch gekennzeichnet, dass** 0,1 bis 10 Gew.- % bevorzugt 0,2 bis 5 Gew.- % des eingesetzten Wasserstoffs ausgeschleust werden und darin die Konzentration des einen oder der mehreren gasförmigen Nebenprodukte bestimmt wird.

5. Verfahren nach einem der vorstehenden Ansprüche, **dadurch gekennzeichnet, dass** die Konzentration des gasförmigen Nebenproduktes über eine Gas-Sonde oder spektroskopisch untersucht wird, insbesondere UV/VIS- oder IR-spektroskopisch.

6. Verfahren nach einem der vorstehenden Ansprüche, **dadurch gekennzeichnet, dass** vor der Bestimmung der Konzentration des gasförmigen Nebenproduktes in der Gasphase enthaltener Wasserdampf durch Kondensation entfernt wird.

7. Verfahren nach einem der vorstehenden Ansprüche, **dadurch gekennzeichnet, dass** das gasförmige Nebenprodukt vor der Konzentrationsbestimmung durch eine Trenneinrichtung geführt wird, insbesondere durch eine chromatographische Säule, vorzugsweise eine GC-Säule.

8. Verfahren nach einem der vorstehenden Ansprüche, **dadurch gekennzeichnet, dass** verbrauchter Katalysator kontinuierlich oder schrittweise ausgeschleust wird.

9. Verfahren nach einem der vorstehenden Ansprüche, **dadurch gekennzeichnet, dass** die organische Nitroverbindung durch Nitrierung einer organischen Verbindung insbesondere mit NOₓ und/ oder Salpetersäure erzeugt wird, wobei die organische Verbindung vorzugsweise ausgewählt ist aus aromatischen Kohlenwasserstoffen, insbesondere Toluol.

10. Verfahren nach einem der vorstehenden Ansprüche, **dadurch gekennzeichnet, dass** die organische Nitroverbindung, Dinitrotoluol (in Formel (I) R2 = Methyl und R3 = NO₂) ist, das zu Toluylendiamin (in Formel (II) R1 = NH₂ und R2 = Methyl) hydriert wird.

11. Verfahren nach einem der vorstehenden Ansprüche, **dadurch gekennzeichnet, dass** die Hydrierung bei einer Temperatur von 80 bis 200 °C durchgeführt wird, insbesondere bei 110 bis 180 °C und / oder dass die Hydrierung bei einem Druck von 5 bis 120 bar durchgeführt wird, insbesondere bei 10 bis 100 bar.

## Claims

1. Method for producing organic amino compounds of formula (II) 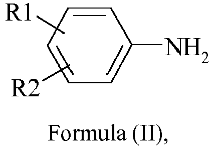 in which R1 and R2 independently of each other denote hydrogen, methyl or ethyl, wherein R1 can additionally denote NH₂, from organic nitro compounds of formula (I) 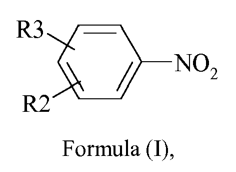 in which R2 and R3 independently of each other denote hydrogen, methyl or ethyl, wherein R3 can additionally denote NO₂, wherein the organic nitro compound is hydrogenated to the organic amino compound with a hydrogen-containing gas stream by means of a catalyst, wherein the reaction course of the hydrogenation is monitored by analysis of secondary products forming during hydrogenation, wherein the concentration of one or more gaseous secondary products is determined in the gas phase and if the concentration of the one or more gaseous secondary products falls below a predefinable minimum concentration, the hydrogenating activity of the catalyst is increased by increasing the volumetric flow rate of the hydrogen-containing gas stream, by throttling the feed of organic nitro compound, by increasing the pressure, by increasing the temperature, by increasing the residence time and/or by adding further catalyst.

2. Method according to claim 1, wherein the gaseous secondary product has a boiling point under normal pressure of 200°C or less, in particular of 180°C or less, more preferably of 80°C or less, particularly preferably of 50°C or less, most particularly preferably of 20°C or less.

3. Method according to claim 1 or 2, wherein the gaseous secondary product is methane, ammonia and/or an aliphatic amine which is not itself one of the products, such as methylamine and/or methylcyclohexylamine, the preferred minimum concentration of the gaseous secondary product
in the case of methane being at least 2 mg/kg of organic nitro compound, preferably 2 to 20, in particular 2 mg/kg, preferably 4 mg/kg, more preferably 10 mg/kg, or even 15 mg/kg,
in the case of ammonia being at least 2 mg/kg of organic nitro compound, preferably 2 to 20 mg/kg, in particular 2 mg/kg, preferably 4 mg/kg, more preferably 10 mg/kg, or even 15 mg/kg, and
in the case of the aliphatic amine being at least 1 mg/kg of organic nitro compound, preferably 1 to 10 mg/kg, in particular 1 mg/kg, preferably 2 mg/kg, more preferably 5 mg/kg, or even 7.5 mg/kg.

4. Method according to any one of the preceding claims, wherein 0.1 to 10 wt.%, preferably 0.2 to 5 wt.%, of the hydrogen that is used is discharged and the concentration of the one or more gaseous secondary products therein is determined.

5. Method according to any one of the preceding claims, wherein the concentration of the gaseous secondary product is analysed by means of a gas probe or by spectroscopy, in particular by UV/VIS or IR spectroscopy.

6. Method according to any one of the preceding claims, wherein water vapor present in the gas phase is removed by condensation before determining the concentration of the gaseous secondary product.

7. Method according to any one of the preceding claims, wherein the gaseous secondary product is passed through a separating apparatus, in particular through a chromatographic column, preferably a GC column, prior to determining the concentration.

8. Method according to any one of the preceding claims, wherein spent catalyst is continuously or gradually discharged.

9. Method according to any one of the preceding claims, wherein the organic nitro compound is produced by nitration of an organic compound in particular with NOₓ and/or nitric acid, wherein the organic compound is preferably selected from aromatic hydrocarbons, in particular toluene.

10. Method according to any one of the preceding claims, wherein the organic nitro compound is dinitrotoluene (in formula (I) R2 = methyl and R3 = NO₂), which is hydrogenated to toluylene diamine (in formula (II) R1 = NH₂ and R2 = methyl).

11. Method according to any one of the preceding claims, wherein the hydrogenation is performed at a temperature from 80 to 200°C, in particular at 110 to 180°C, and/or the hydrogenation is performed under a pressure from 5 to 120 bar, in particular at 10 to 100 bar.

## Revendications

1. Procédé pour la préparation de composés amino organiques de formule (II) 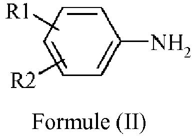 dans laquelle R1 et R2 représentent, indépendamment l'un de l'autre, hydrogène, méthyle ou éthyle, R1 pouvant en outre signifier NH₂, à partir de composés nitro organiques de formule (I) 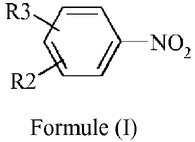 dans laquelle R2 et R3 représentent, indépendamment l'un de l'autre, hydrogène, méthyle ou éthyle, R3 pouvant en outre signifier NO₂, le composé nitro organique étant hydrogéné en composé amino organique à l'aide d'un flux gazeux contenant de l'hydrogène au moyen d'un catalyseur, l'évolution de la réaction d'hydrogénation étant surveillée par analyse de produits secondaires formés lors de l'hydrogénation, **caractérisé en ce que** la concentration en un ou plusieurs produits secondaires gazeux dans la phase gazeuse est déterminée et lors du passage sous une concentration minimale pouvant être prédéfinie en ledit un ou lesdits plusieurs produits secondaires gazeux, l'activité d'hydrogénation du catalyseur est augmentée par augmentation du flux volumique du flux gazeux contenant de l'hydrogène, par étranglement de l'alimentation en composé nitro organique, par augmentation de la pression, par augmentation de la température, par augmentation du temps de séjour et/ou par addition de catalyseur supplémentaire.

2. Procédé selon la revendication 1, **caractérisé en ce que** le produit secondaire gazeux présente un point d'ébullition à pression normale de 200°C ou moins, en particulier de 180°C ou moins, plus préférablement de 80°C ou moins, de manière particulièrement préférée de 50°C ou moins de manière tout particulièrement préférée de 20°C ou moins.

3. Procédé selon la revendication 1 ou 2, **caractérisé en ce que** le produit secondaire gazeux est le méthane, l'ammoniac et/ou une amine aliphatique, qui ne compte pas elle-même parmi les produits, telle que la méthylamine et/ou la méthylcyclohexylamine, la concentration minimale préférée en produit secondaire gazeux,
dans le cas du méthane, étant d'au moins 2 mg/kg de composé nitro organique, de préférence de 2 à 20, en particulier de 2 mg/kg, de préférence de 4 mg/kg, plus préférablement de 10 mg/kg ou même de 15 mg/kg,
dans le cas de l'ammoniac, étant d'au moins 2 mg/kg de composé nitro organique, de préférence de 2 à 20 mg/kg, en particulier de 2 mg/kg, de préférence de 4 mg/kg, plus préférablement de 10 mg/kg ou même de 15 mg/kg, ainsi que
dans le cas de l'amine aliphatique, étant d'au moins 1 mg/kg de composé nitro organique, de préférence de 1 à 10 mg/kg, en particulier de 1 mg/kg, de préférence de 2 mg/kg, plus préférablement de 5 mg/kg ou même de 7,5 mg/kg.

4. Procédé selon l'une quelconque des revendications précédentes, **caractérisé en ce que** 0,1 à 10% en poids, de préférence 0,2 à 5% en poids de l'hydrogène utilisé sont évacués et on y détermine la concentration en ledit un ou lesdits plusieurs produits secondaires gazeux.

5. Procédé selon l'une quelconque des revendications précédentes, **caractérisé en ce que** la concentration en produit secondaire gazeux est analysée via une sonde gazeuse ou par spectroscopie, en particulier par spectroscopie UV/VIS ou IR.

6. Procédé selon l'une quelconque des revendications précédentes, **caractérisé en ce qu'**avant la détermination de la concentration en produit secondaire gazeux, la vapeur d'eau contenue dans la phase gazeuse est éliminée par condensation.

7. Procédé selon l'une quelconque des revendications précédentes, **caractérisé en ce que** le produit secondaire gazeux est guidé à travers un dispositif de séparation avant la détermination de la concentration, en particulier à travers une chromatographie, de préférence une colonne de CG.

8. Procédé selon l'une quelconque des revendications précédentes, **caractérisé en ce que** le catalyseur usagé est évacué en continu ou par lots.

9. Procédé selon l'une quelconque des revendications précédentes, **caractérisé en ce que** le composé nitro organique est produit par nitruration d'un composé organique, en particulier avec du NOₓ et/ou de l'acide nitrique, le composé organique étant de préférence choisi parmi les hydrocarbures aromatiques, en particulier le toluène.

10. Procédé selon l'une quelconque des revendications précédentes, **caractérisé en ce que** le composé nitro organique est le dinitrotoluène (dans la formule (I) R2 = méthyle et R3 = NO₂), qui est hydrogéné en toluylènediamine (dans la formule (II) R1 = NH₂ et R2 = méthyle).

11. Procédé selon l'une quelconque des revendications précédentes, **caractérisé en ce que** l'hydrogénation est réalisée à une température de 80 à 200°C, en particulier de 110 à 180°C et/ou **en ce que** l'hydrogénation est réalisée à une pression de 5 à 120 bars, en particulier de 10 à 100 bars.
